# EUROPÄISCHE PATENTSCHRIFT

(11) **EP 1 067 126 B1**
(45) Veröffentlichungstag und Bekanntmachung des Hinweises auf die Patenterteilung: **21.12.2005**
(21) Anmeldenummer: 00113135.8
(22) Anmeldetag: 29.06.2000
(51) Int. Cl.: C07D 307/93, A61K 7/46, C11B 9/00

(54) **Tetracyclische Acetale**
Tetracyclic acetals
Acétales tétracycliques

(30) Priorität: 08.07.1999 DE 19931709
(43) Veröffentlichungstag der Anmeldung: 10.01.2001
(73) Patentinhaber: Symrise GmbH & Co. KG, 37603 Holzminden (DE)
(72) Erfinder: Surburg, Horst, Dr., 37603 Holzminden (DE); Wörner, Peter, 37603 Holzminden (DE)
(74) Vertreter: Eisenführ, Speiser & Partner

(56) Entgegenhaltungen:
- EP-A- 0 857 723
- WO-A-92/05166
- B.A. MCANDREW ET AL.: "The Acetylation of Cedrene in the Presence of Titanium Tetrachloride" JOURNAL OF THE CHEMICAL SOCIETY, PERKIN TRANSACTIONS 1, 1983, Seiten 1373-1378, XP002167148 LETCHWORTH GB

## Beschreibung

Trotz einer Vielzahl bereits vorhandener Riechstoffe besteht in der Parfümindustrie weiterhin Bedarf an neuen Riechstoffen, die über ihre geruchlichen Eigenschaften hinaus zusätzliche positive Sekundäreigenschaften haben, wie z.B. höhere Stabilität, höhere Ausgiebigkeit, besseres Haftungsvermögen usw. aurweisen

Ein erster Aspekt der vorliegenden Erfindung betrifft tetracyclische Acetale gemäß Patentanspruch 1. Insbesondere wurden neue tetracyclische Acetale der beiden isomeren Strukturen 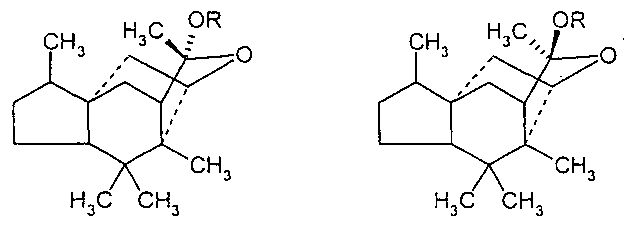 gefunden, worin
- R: Niederalkyl bedeutet.

Diese Acetale eignen sich in besonderem Maße als Riechstoffe, die in Parfümierungen eingesetzt werden können.

Niederalkyl steht hierbei für Methyl, Ethyl, n-Propyl, iso-Propyl, n-Butyl oder iso-Butyl.

Insbesondere bevorzugt sind Methyl und Ethyl.

Die Herstellung der neuen tetracyclischen Acetale erfolgt auf an sich bekannte Weise ausgehend von alpha-Cedren, das mit Essisäureanhydrid in Gegenwart von Titantetrachlorid als einer stärkeren Lewis-Säure umgesetzt wird. Dabei bildet sich nicht wie bei der Umsetzung mit sauren Katalysatoren wie z. B. Phosphorsäure das sog. "Acetylcedren" ("Methylcedrylketon"), ein Riechstoff, der bei der Herstellung von Parfüms seit langem eine breite Verwendung findet, sondern unter Gerüstumlagerung und Wasseranlagerung das tricyclische Hydroxyketon 1-(Octahydro-8-hydroxy-3,6,7,7-tetramethyl-3a,6-ethano-3aH-inden-5-yl)-ethanon, welches beim Erhitzen unter Abspaltung von Wasser leicht in den tetracyclischen Enolether Decahydro-2-methylen-3a,4,4,7-tetramethyl-3,7a-methano-7aH-indeno[5,6-b]furan übergeht. Unter Anlagerung von Wasser an den Enolether bildet sich leicht unter Ringöffnung das Hydroxyketon zurück (s.a. J. Chem. Soc. Perkin Trans. I **1983**, S. 1373-78).

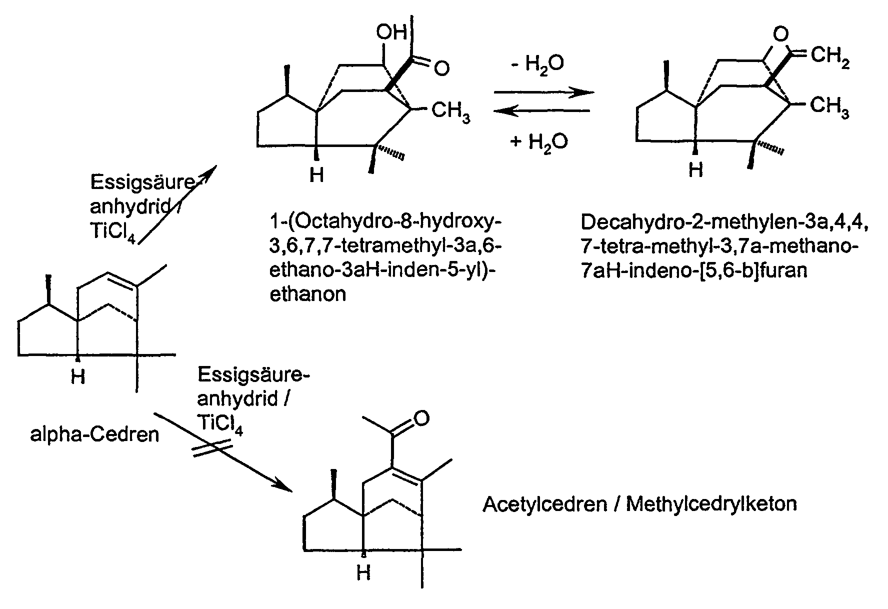

Das Hydroxyketon ist in reiner Form geruchlos; der Enolether hat lediglich einen schwachen an die Sesquiterpenkohlenwasserstoff-Fraktion von Cedernholzöl ("Cedernholzöl-Terpene") erinnernden Geruch.

Überraschenderweise wurde nun gefunden, dass alkoholische Lösungen sowohl des Hydroxyketons als auch Enolethers einen starken charakteristischen Holzgeruch zeigen, der den beiden reinen Verbindungen nicht zueigen ist. Wir fanden, dass die Ursache hierfür in der Bildung von neuen, bisher noch nicht beschriebenen tetracyclischen Acetalen liegt. Die Bildung dieser Acetale wird durch die folgenden beiden Formelschemata beschrieben:

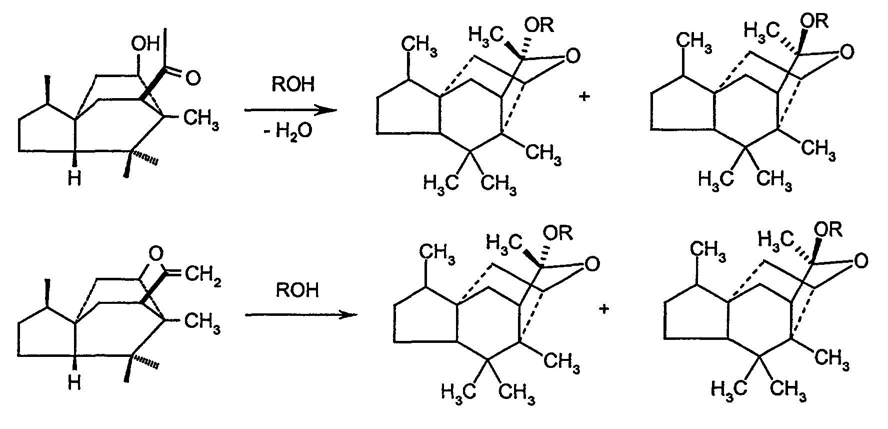

Die erfindungsgemäßen Acetale liegen in der Regel als Gemisch zweier Isomere vor.

Beide Isomere weisen einen ausgeprägten intensiven und ungewöhnlich lang haftenden, frischen Holzgeruch auf.

Wegen dieser besonderen organoleptischen Eigenschaften eignen sich diese Acetale in hervorragendem Maße für die Verwendung als Riechstoffe.

Die Herstellung der erfindungsgemäßen Acetale kann im Einzelnen wie folgt durchgeführt werden:

Das Hydroxyketon 1-(Octahydro-8-hydroxy-3,6,7,7-tetramethyl-3a,6-ethano-3aH-inden-5-yl)-ethanon, oder der Enolether Decahydro-2-methylen-3a,4,4,7-tetramethyl-3,7a-methano-7aH-indeno[5,6-b]furan oder ein Gemisch von beiden wird mit einem niederen Alkohol versetzt, wobei am günstigsten der betreffende Alkohol im Überschuss eingesetzt wird und gleichzeitig als Lösungsmittel dient. Es kann aber auch ein anderes inertes Lösungsmittel zugesetzt werden. Die Reaktion kann in einem weiten Temperaturbereich erfolgen, z. B. zwischen -25° und +150°C, ggf. unter Druck. Bevorzugt ist ein Temperaturbereich zwischen 20° und 80°C.

Nach Abdestillieren des überschüssigen Alkohols, bzw. anderer Lösungsmittel werden die erfindungsgemäßen Acetale als Rückstand erhalten; ggf. kann auch die alkoholische Lösung direkt weiterverwendet werden.

Die erfindungsgemäßen Acetale können dabei als Einzelstoffe in einer Vielzahl von Produkten verwendet werden; besonders vorteilhaft lassen sie sich mit anderen Riechstoffen zu neuartigen Parfümkompositionen kombinieren.

Durch die Verwendung der erfindungsgemäßen Acetale lassen sich in der Regel bereits in geringer Dosierung in den resultierenden Parfümkompositionen strahlend frische, holzige Geruchsnoten erzielen, wobei der geruchliche Geamteindruck auffallend harmonisiert, die Austrahlung wahrnehmbar erhöht und die Fixierung, d.h. das Haftvermögen des Parfümöles, deutlich verstärkt wird.

Beispiele für Riechstoffe, mit denen die erfindungsgemäßen Acetale vorteilhaft kombiniert werden können, finden sich z.B. in *S*. *Arctander*, *Perfume and Flavor Materials*, *Vol*. *I und II*, *Montclair*, *N*. *J*., *1969*, *Selbstverlag* oder *K*. *Bauer*, *D*. *Garbe und H*. *Surburg*, *Common Fragrance and Flavor Materials*, *3*^{*rd*}. *Ed*., *Wiley-VCH*, *Weinheim 1997*.

Im einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methyl-2-heptanol, 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinoat; Methyl-2-noninoat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavandulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpinen-ol-4; Menthan-8-ol; Menthan-1-ol; Menthan-7-ol; Bomeol; Isobomeol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha-3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert.-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl- 2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaldehyddimethylacetal; Phenylacetaldehydglycerinacetal; 2,4,6-Trimethyl-4-phenyl-1,3-dioxan; 4,4a,5,9b-Tetrahydro-indeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8',hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methyl-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die die erfindungsgemäßen Acetale enthaltenden Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können die die erfindungsgemäßen Acetale enthaltenden Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die die erfindungsgemäßen Acetale enthaltenden Parfümöle können auch mikroverkapselt, sprühgetrocknet, als Einschluß-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erhalten werden.

In Parfümkompositionen beträgt die eingesetzte Menge der erfindungsgemäßen Acetale 0,05 bis 50 Gew.-%, vorzugsweise 0,5 bis 20 %, bezogen auf das gesamte Parfümöl.

Die die erfindungsgemäßen Acetale enthaltenden Parfümöle können in konzentrierter Form, in Lösungen oder in oben beschriebener modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, Aftershave-Cremes und -lotionen, Bräunungscremes und -lotionen, Haarpflegeprodukten wie z.B. Haarsprays, Haargelen, festigende Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Ein Schwerpunkt der Verwendung der erfindungsgemäßen Acetale liegt wegen ihrer Stabilität im alkalischen Bereich bei der Parfümierung von Seifen und Waschmitteln. Bei der Verwendung in Waschmittelparfümierungen zeichnen sich die erfindungsgemäßen Acetale durch eine im Vergleich zu bisher verwendeten Riechstoffen erhöhte Substantivität, d.h. durch ein verstärktes Aufziehvermögen und eine erhöhte Haftung des Riechstoffs auf der gewaschenen Faser, aus.

Bei der Parfümierung der beschriebenen Produkte kann die Menge der ParfümKomposition 0,1 bis 40 Gew.-%, vorzugsweise 0,5 bis 20 Gew.-%, bezogen auf das ganze Produkt, betragen.

### Beispiele

### Beispiel 1:

### Herstellung einer Parfümkomposition zur Verwendung in einem Eau-de-Toilette für Herren:

Es werden vermischt (alle Angaben als Gewichtsteile):

| | |
|---|---|
| Agrumex HC H&R | 20 |
| Ambroxid H&R | 5 |
| Benzylsalicylat | 150 |
| Citral | 15 |
| Citrylal H&R | 5 |
| CPD supra H&R 50% in TEC* | 20 |
| Cumarin | 10 |
| Dihydromyrcenol | 100 |
| Evernyl Givaudan-Roure | 5 |
| Firbalsam Absolue | 15 |
| Freesiol H&R | 15 |
| Geranitnl H&R | 5 |
| Hedion Firmenich | 40 |
| Hexylsalicylat | 25 |
| Isoananat H&R | 5 |
| Isobornylacetat | 55 |
| Lavandinöl Grosso | 20 |
| Lilial Givaudan-Roure | 10 |
| Linalool | 75 |
| Linalylacetat | 100 |
| Orangenöl weiß | 100 |
| Patchoulyöl farbl. | 10 |
| Sandolen H&R | 10 |
| Vertocitral H&R | 5 |

| | |
|---|---|
| * Triethylcitrat | |

a) Durch Zugabe von 30 g Decahydro-2-ethoxy-2,3a,4,4,7-pentamethyl-3,7a-methano-7aH-indeno[5,6-b]furan wird die masculine Frische des Parfüms pudrig-holzig unterlegt und der Duft holzig fixiert.
b) Durch Zugabe von 80 g Decahydro-2-ethoxy-2,3a,4,4,7-pentamethyl-3,7a-methano-7aH-indeno[5,6-b]furan wird die Parfümkomposition betont holzig und die Fixierung wird deutlich verstärkt.

### Beispiel 2:

### Herstellung von Decahydro-2-methoxy-2,3a,4,4,7-pentamethyl-3,7a-methano-7aH-indeno[5,6-b]furan (Isomerengemisch der tetracyclischen Acetale mit R = Methyl):

10 g Decahydro-2-methylen-3a,4,4,7-tetramethyl-3,7a-methano-7aH-indeno[5,6-b]furan (hergestellt aus alpha-Cedren und Essigsäureanhydrid in Gegenwart von Aluminiumtrichlorid analog J. Chem. Soc. Perkin Trans. I **1983**, S. 1373-78) wurden in 100 g Methanol gelöst und 48 h bei Raumtemperatur stehengelassen. Nach Zugabe von etwas Bicarbonat wurde das Methanol bei einer Temperatur von max. 30° abgezogen. Als Rückstand verblieb ein Gemisch der beiden isomeren Acetale mit R = Methyl. Zur Strukturbestimmung wurden die Isomeren durch Flüssig-Chromatographie an basischem Aluminiumoxid getrennt.

¹H-NMR-Spektrum (400 MHz; C₆D₆; TMS = 0 ppm):
Isomer A:
   3,85 (dd, J = 6.6, 1.3, 1H); 3,83 (s, 3H, O-CH3); 1,51 (s, 3H); 1,1 (s, 3H); 0,94 (s, 3H); 0,84 (d, J = 7.3; 3H); 0,75 (s, 3H).
Isomer B:
   3,81 (dd, J = 7.4, 1.5; 1H); 3,30 (s, 3H, O-CH3); 1,41 (s, 3H); 1,40 (s, 3H); 0,96 (s, 3H); 0,77 (s, 3H); 0,73 (d, J = 7.3; 3H).
Massenspektrum:
   Isomer A: 263 (4, M⁺-15);246 (30); 204 (18); 161(26); 119(100); 43(54).
   Isomer B: 263 (4, M⁺-15);246 (20); 204 (26); 161(34); 119(100); 43(48).

### Beispiel 3:

### Herstellung von Decahydro-2-ethoxy-2,3a,4,4,7-pentamethyl-3,7a-methano-7aH-indeno[5,6-b]furan (Isomerengemisch der tetracyclischen Acetale mit R = Ethyl):

5 g 1-(Octahydro-8-hydroxy-3,6,7,7-tetramethyl-3a,6-ethano-3aH-inden-5-yl)-ethanon (hergestellt aus alpha-Cedren und Essigsäureanhydrid in Gegenwart von Aluminiumtrichlorid analog J. Chem. Soc. Perkin Trans. I **1983**, S. 1373-78) wurden mit 50 g Ethanol versetzt und 10 min am Rückfluß gekocht. Nach Abkühlen wurde etwas Bicarbonat hinzugefügt und das Ethanol bei einer Temperatur von max. 30° abgezogen.

Als Rückstand verblieb ein Gemisch der beiden isomeren Acetale mit R = Ethyl. Zur Strukturbestimmung wurden die Isomeren durch Flüssig-Chromatographie an basischem Aluminiumoxid getrennt.

¹H-NMR-Spektrum (400 MHz; C₆D₆; TMS = 0 ppm):
Isomer A:
   3,83 (qd, J = 9.2, 7.1; 1H); 3,53 (qd, J = 9.2, 7.0; 1H);1,45 (s, 3H); 1,42 (s, 3H); 1,19 (t, J = 7,1; 3H); 0,74 (d, J = 7.3; 3H); 0,98 (s, 3H); 0,78 (s, 3H).
Isomer B:
   3,91 (qd, J = 9.2, 7.1; 1H); 3,55 (qd, J = 9.2, 7.0; 1H); 1,54 (s, 3H); 1,11 (s, 3H); 1,19 (t, J = 7,1; 3H); 0,87 (d, J = 7.3; 3H); 0,95 (s, 3H); 0,76 (s, 3H).
Massenspektrum:
   Isomer A: 277 (3, M⁺-15);246 (22); 204 (18); 161(27); 119(100); 43(62).
   Isomer B: 277 (3, M⁺-15);246 (14); 204 (22); 161(38); 119(100); 43(60).

## Patentansprüche

1. 2-Alkoxydecahydro-2,3a,4,4,7-pentamethyl-3,7a-methano-7aH-indeno[5,6-b]furane,
worin
R eine geradkettige oder verzweigte Alkyl- oder Alkenylgruppe mit 1 bis 5 C-Atomen und einer Doppelbindung bedeutet.

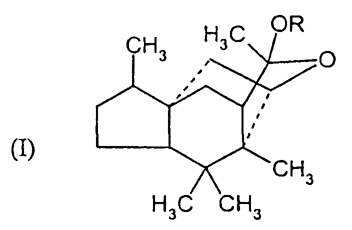

2. Verfahren zur Herstellung von 2-Alkoxydecahydro-2,3a,4,4,7-pentamethyl-3,7a-methano-7aH-indeno[5,6-b]furanen gemäß Patentanspruch 1, durch Umsetzung von Decahydro-2-methylen-3a,4,4,7-tetramethyl-3,7a-methano-7aH-indeno[5,6-b]furan oder 1-(Octahydro-8-hydroxy-3,6,7,7-tetramethyl-3a,6-ethano-3aH-inden-5-yl) ethanon mit Alkoholen.

3. Verwendung von 2-Alkoxydecahydro-2,3a,4,4,7-pentamethyl-3,7a-methano-7aH-indeno[5,6-b]furanen gemäß Anspruch 1 als Riechstoffe.

4. Parfümöle enthaltend 2-Alkoxydecahydro-2,3a,4,4,7-pentamethyl-3,7a-methano-7aH-indeno[5,6-b]furanen gemäß Anspruch 1.

## Claims

1. 2-Alkoxydecahydro-2, 3a, 9, 9, 7-pentamethyl-3,7a-methano-7aH-indeno[5,6-b]furans,
wherein
R denotes a straight-chain or branched alkyl or alkenyl group having 1 to 5 C atoms and a double bond.

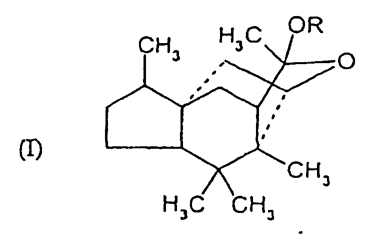

2. Process for preparing 2-alkoxydecahydro-2,3a,9,9,7-pentamethyl-3,7a-methano-7aH-indeno[5,6-b]furans according to claim 1, by reacting decahydro-2-methylene-3a,4,4,7-tetramethyl-3,7a-methano-7aH-indeno[5,6-b]furan or 1-(octahydro-8-hydroxy-3,6,7,7-tetramethyl-3a,6-ethano-3aH-inden-5-yl) ethanone with alcohols.

3. Use of 2-alkoxydecahydro-2,3a,4,4,7-pentamethyl-3,7a-methano-7aH-indeno[5,6-b]furans according to claim 1 as fragrance substances.

4. Perfume oils containing 2-alkoxydecahydro-2,3a,4,4,7-pentamethyl-3,7a-methano-7aH-indeno[5,6-b]furans according to claim 1.

## Revendications

1. 2-Alcoxydécahydro-2,3a,4,4,7-pentaméthyl-3,7a-méthano-7aH-indéno[5,6-b]furanes, dans lesquels
R représente un groupe alkyle ou alcényle linéaire ou ramifié contenant 1 à 5 atomes de carbone et une double liaison.

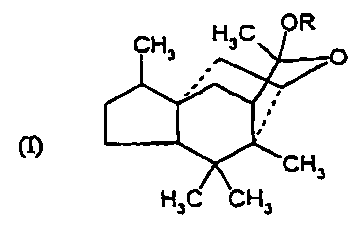

2. Procédé de préparation de 2-alcoxydécahydro-2,3a,4,4,7-pentaméthyl-3,7a-méthano-7aH-indéno[5,6-b]furanes selon la revendication 1 par réaction de décahydro-2-méthylène-3a,4,4,7-tétraméthyl-3,7a-méthano-7aH-indéno[5,6-b]furane ou de 1-(octahydro-8-hydroxy-3,6,7,7-tétraméthyl-3a,6-éthano-3aH-indén-5-yl)éthanone avec des alcools.

3. Utilisation de 2-alcoxydécahydro-2,3a,4,4,7-pentaméthyl-3,7a-méthano-7aH-indéno[5,6-b]furanes selon la revendication 1 comme produits odorants.

4. Essences de parfums contenant des 2-alcoxydécahydro-2,3a,4,4,7-pentaméthyl-3,7a-méthano-7aH-indéno[5,6-b]furanes selon la revendication 1.
